# FASCICULE DE BREVET EUROPEEN

(11) **EP 2 011 452 B1**
(45) Date de publication et mention de la délivrance du brevet: **30.04.2014**
(21) Numéro de dépôt: 07447042.8
(22) Date de dépôt: 06.07.2007
(51) Int. Cl.: A61L 27/50, A61L 27/54, A61L 27/58, A61K 47/14, A61F 2/08, A61K 9/00

(54) **Préparation injectable pour la réalisation d'un implant musculaire**
Injizierbare Zubereitung zum Erhalten eines Muskelimplantat
Injectable preparation for obtaining a muscle implant

(43) Date de publication de la demande: 07.01.2009
(73) Titulaire: S.B.I.R. SPRL, 5100 Jambes (BE)
(72) Inventeur: Hebrant, Jean, B-5100 Jambes (BE); Lidarssi, Terry, B-1040 Bruxelles (BE); De Schryver, Christophe, B-4053 Embourg (BE); Kress, Robert Karl, D-24103 Kiel (DE)
(74) Mandataire: Pronovem

(56) Documents cités:
- WO-A-96/39995
- US-A- 5 709 873
- MARTEN ET AL: "Medium-chain triglycerides" INTERNATIONAL DAIRY JOURNAL, ELSEVIER APPLIED SCIENCE, BARKING,, GB, vol. 16, no. 11, novembre 2006 (2006-11), pages 1374-1382, XP005671509 ISSN: 0958-6946

## Description

### Objet de l'invention

La présente invention se rapporte à une préparation ou composition injectable en intramusculaire et bio-résorbable pour la réalisation d'un implant musculaire.

Le domaine d'application de l'invention est le domaine esthétique (implants fessiers, bodybuilding, etc.) et le domaine médical (reconstruction dans le cas de maladies neurodégénératives, traitement des séquelles de la polyomélite, du SIDA, etc.).

### Etat de la technique

Actuellement, dans le domaine de l'esthétique, la demande pour les implants fessiers, permettant de donner un galbe et une jolie forme aux fesses, après être apparue d'abord en Amérique du Sud, s'est internationalisée et est devenue de plus en plus importante. Toutefois, contrairement au cas des implants mammaires, le taux de réussite est très faible (infection, rejet, etc.).

On connaît ces implants sous forme d'une enveloppe en élastomère de silicone contenant du sérum physiologique ou du gel de silicone cohésif. Dans le premier cas, il y a risque important de rupture, ces implants étant beaucoup plus sollicités que les implants mammaires par exemple ; dans le second cas, une marque et un tissu fibrotique irréversible apparaissent entre le grand fessier et le moyen fessier.

D'autre part, on sait que les triglycérides à chaînes moyennes (MCT) sont obtenus par estérification avec du glycérol d'acides gras comprenant généralement entre 6 et 14 atomes de carbone. Par exemple, des émulsions huileuses végétales de MCT, disponibles dans le commerce, sont obtenues par fractionnement d'huile de noix de coco et/ou d'huile de noyaux de palme. Cette huile contient presque exclusivement de l'acide caprylique ou octanoïque (C8) et de l'acide caprique ou décanoïque (C10). L'aspect de cette huile est incolore à jaune pâle, d'odeur et de goût neutre. Les MCT sont 100 fois plus solubles dans l'eau que les triglycérides à chaînes longues (LCT), et, à ce titre, conviennent pour une injection. De plus,il est connu que les acides gras à chaînes moyennes sont plus rapidement absorbés et oxydés que les longues chaînes d'acides gras. De plus des études sur les rats ont montré que les régimes alimentaires avec chaînes moyennes de triglycérides augmentent les dépenses énergétiques (effet thermique augmenté) tout en provoquant un stockage adipeux moindre.

Les MCT sont ainsi utilisés par ingestion ou par injection parentérale, le plus souvent intraveineuse, chez des individus, malades ou sportifs, requérant un supplément nutritionnel ou énergétique (par exemple Traul, K.A., et al., Review of the Toxicologic Properties of Medium-chain Triglycérides, Food Chem. Toxicol. 38(2000), p. 79-98) ou comme excipient de composition thérapeutique, par exemple pour des produits lipophiles injectables (par exemple WO-A-90/14094). Dans ce cas, les MCT ont un rôle de support inerte de substance active ou un rôle de support favorisant la diffusion de la substance active.

L'information disponible sur l'absorption et le métabolisme des MCT suggère que les MCT injectés peuvent être absorbés par la circulation sanguine et transportés vers le foie en vue de leur métabolisme et leur destruction.

Aucune réaction musculaire, myonécrose ou autres symptômes toxicologiques n'ont été rapportés suite à l'administration de 0,5 mL MCT chez le lapin. Avec des injections répétées de 0,5 mL MCT chez le lapin pendant 14 jours, on n'a relevé aucuns signes cliniques, pathologiques macroscopiques ou microscopiques anormaux, à l'exception de kystes et de granulomatomes huileux dans les tissus intersticiels au site d'injection.

De même, les MCT ne sont pas ou sont peu considérés comme irritants dermiques ou oculaires, ni comme sensibilisateurs et n'induisent pas de photosensibilisation.

Les MCT interagissent peu avec le système immunitaire, sont très peu toxiques et ne présentent pas de génotoxicité. Cependant, les MCT sont contrindiqués chez les patients présentant une encéphalopathie hépatique ou des fonctions métaboliques altérées (cirrhose, diabète, insuffisance rénale, etc.).

Des implants de MCT ont été proposés comme une alternative sûre aux implants mammaires au silicone. Une étude a démontré que les triglycérides qui pourraient s'échapper des implants sont absorbés et métabolisés. Ils sont alors redistribués vers les réserves naturelles de graisse dans l'organisme ou leurs métabolites sont excrétés, principalement dans l'urine. Aucune réponse allergique ou toxique n'a été rapportée après implantation ou injection. Des expériences in vitro ont montré que les bactéries associées habituellement aux infections par blessure ne croissent pas dans les triglycérides.

L'alcool benzylique est également utilisé largement dans beaucoup de compositions injectables, qu'elles soient intraveineuses ou intramusculaires, pour ses propriétés de solvant et de conservateur dans les produits injectables multi-doses. Le métabolisme de l'alcool benzylique en alcool benzoïque chez l'homme est bien connu. Les effets biochimiques en ont été investigués dans des études spécifiques. La toxicité a été étudiée de manière exhaustive, en ce compris la toxicité aiguë, court terme et long terme, la carcinogénicité, la génotoxicité et la toxicité développementale. Le Comité Scientifique sur la Nourriture (Commission Européenne) considère que les études sur la carcinogénicité chez les rats et souris n'ont pas montré d'effets contraires liés aux composés en question à des niveaux de dose allant jusqu'à 200 mg/kg chez la souris (respectivement 400 mg/kg chez le rat). L'alcool benzylique a cependant été trouvé en association avec le collapse cardiovasculaire néonatal (« gasping baby syndrome ») en 1982. En conséquence, tant la FDA que l'Académie Américaine de Pédiatrie recommandent l'arrêt de son utilisation chez l'enfant lorsque c'est possible.

L'alcool benzylique est également potentiellement toxique lorsqu'il est administré localement à un tissu nerveux. Par exemple, on rapporte des cas de séquelles neurologiques liées à une administration épidurale par inadvertance. L'alcool benzylique a aussi été impliqué dans le développement de paraparésie en utilisation de diluant pour régime de chimiothérapie intrathécale avec du méthotrexate ou de la cytarabine. Des études menées sur l'animal suggèrent que la neurotoxicité de l'acide benzylique dans l'espace intrathécal pourrait être reliée à la concentration, au volume et au temps d'exposition. Bien que la preuve de neurotoxicité à des concentrations utilisées cliniquement (0,9%) est quelque peu circonstancielle, la prudence impose de ne pas administrer l'alcool benzylique intraspinellement.

Le tocophérol (vitamine E) est également largement utilisé dans beaucoup de produits injectables, de manière soit intraveineuse, soit intramusculaire. C'est un agent antioxydant. Aucune toxicité n'a été mise en évidence, même à très forte dose.

### Buts de l'invention

La présente invention vise à fournir une solution qui permet d'éviter les inconvénients de l'état de la technique.

En particulier, l'invention a pour objet de proposer un implant musculaire qui soit bien toléré par le corps, non toxique, sans danger et irréprochable du point de vue esthétique.

### Résumé de l'invention

L'objet de la présente invention se rapporte à l'utilisation d'une préparation injectable en intra-musculaire et bio-résorbable dans l'organisme humain ou animal, se présentant sous la forme d'une solution huileuse stérilisée comprenant :
- des triglycérides à chaîne moyenne (MCT), de préférence d'origine végétale ;
- optionnellement de l'alcool benzylique ;
- optionnellement de l'alpha-tocophérol ;
pour la réalisation d'un implant musculaire destiné au traitement de maladies neurodégénératives d'atrophie musculaire ou au traitement des séquelles de la polyomélite ou en vue de l'obtention d'un rééquilibrage ou d'une harmonie musculaire à des fins esthétiques ou à des fins de traitement post-traumatique ou psychologique, ou encore pour la réalisation d'un implant fessier destiné au traitement de la perte musculaire liée au SIDA.

Selon l'invention, lesdits MCT contenus dans ladite préparation sont d'origine végétale et proviennent de l'estérification avec du glycérol d'une composition d'acides gras contenant presque 100% d'acide caprylique (C8) et d'acide caprique (C10).

De préférence, lesdits MCT contenus dans ladite préparation sont d'origine vegétale et proviennent de l'estérification de la composition d'acides gras suivants :
- acide capronique (C6) : < 2% ;
- acide caprylique (C8) : 50 - 80% ;
- acide caprique (C10) : 20 -50% ;
- acide laurique (C12) : < 3% ;
- acide myristique (C14) : < 1%.

Avantageusement, lesdits MCT sont obtenus par fractionnement d'huile de noix de coco et/ou de palme.

Avantageusement encore, la préparation injectable selon l'invention est bio-résorbable en une période de 30 jours.

### Description d'une forme d'exécution préférée de l'invention

La présente invention se rapporte à l'utilisation d'une composition ou préparation injectable en intramusculaire et bio-résorbable, pour réaliser un implant musculaire temporaire ou semi-permanent, contenant un produit liquide huileux, à savoir à base de triglycérides à chaînes moyennes (MCT) d'origine animale ou végétale, mais de préférence d'origine végétale, et le cas échéant d'alcool benzylique et de vitamine E (alpha-tocophérol). Cette préparation se présente de préférence sous la forme de fioles scellées en verre qui ont été soumises, pendant la fabrication, à une filtration aseptique et à un autoclavage.

La composition injectable selon l'invention est destinée à une utilisation dans les cas où l'on souhaite une augmentation du volume musculaire par effet de masse, avec indication esthétique notamment chez des patients souffrant d'atrophie musculaire. L'utilisation de cette préparation injectable sera uniquement active au niveau des muscles striés.

La composition injectée est éliminée par l'organisme. Des injections répétées à une fréquence déterminée sont donc nécessaires pour maintenir l'effet escompté.

La composition injectée selon l'invention ne contient aucun produit pharmaceutique ou pharmacologique actif additionnel.

L'alcool benzylique peut être utilisé dans le cadre de l'invention pour ses propriétés de solvant et de conservateur dans les produits injectables. Le tocophérol peut être utilisé comme agent antioxydant pour améliorer la stabilité de l'implant à l'égard d'éventuels agents oxydants.

Selon une forme d'exécution préférée de l'invention, la composition injectable est présentée sous la forme de fioles de verre scellées contenant chacune 5 mL de MCT, soit pur, soit en solution avec 2,5% poids/vol. (125 mg) d'alcool benzylique et 2,5 % poids/vol. (125 mg) d'acétate d'alpha-tocophérol. Les fioles sont conditionnées dans des boîtes en plastique pouvant contenir 20 unités par exemple. Les composants précités sont bien connus de l'homme de métier et décris dans *European Pharmacopoeia,* 5^{ème} Ed. Selon une variante d'exécution, les fioles peuvent être remplacées par des seringues préremplies.

### Instructions d'utilisation

On évaluera le volume musculaire à augmenter ou à acquérir et on procédera à un historique médical du patient pour s'assurer qu'il n'y a pas de contrindications au traitement.

On nettoiera la peau autour du site d'injection et on séchera celui-ci avant de procéder à l'injection. On injectera la composition selon l'invention en utilisant une seringue et une aiguille stériles.

L'excès de solution, la seringue et l'aiguille usagées seront mis en décharge.

Selon l'effet escompté, 1 à 3 mL sont injectés 1 à 3 fois par semaine jusqu'à ce que ledit effet escompté soit obtenu. Le traitement variera selon le volume musculaire initial, le type de muscle et l'effet escompté.

### Processus intracellulaire

D'une part, 1'injection intramusculaire de MCT conduit à la production d'énergie dans le muscle. En effet, les MCT injectés: dans certains muscles sont métabolisés au sein des cellules endothéliales (capillaires veineux) qui leur sont associées. Les MCT injectés y sont hydrolysés par la lipoprotéine lipase (LPL) en glycérol et acides gras à chaîne moyenne. L'action de la lipoprotéine lipase est activée par l'apolipoprotéine CII. Les produits de décomposition des triglycérides sont transportés par la sérumalbumine vers les mitochondries de la cellule musculaire ou s'effectue le cycle de Krebs (avec *in fine* production d'énergie sous forme d'ATP).

D'autre part, les injectats de MCT ont un effet sur les fibres musculaires du muscle strié (squelettique). Suite à l'injection, les fibres d'actine (filaments minces) et de myosine (filaments épais) s'écartent les unes des autres et une force s'exerce qui leur donne une tendance à reprendre leur place initiale. L'effort auquel ces fibres sont soumises est alors comparable à l'effort fourni par un exercice physique isométrique (c'est-à-dire dans des conditions de maintien du niveau de glycogène du muscle). Par ailleurs, l'aponévrose du muscle, c'est-à-dire l'enveloppe externe de celui-ci, se détend et permet au muscle de prendre du volume, de se développer.

L'injection intra-musculaire provoque un effet de masse lié à un processus inflammatoire modéré. De manière inattendue, ce processus inflammatoire peut avoir une rémanence de plusieurs semaines. On constate que ce phénomène ne se produit pas en l'absence des chaînes d'estérification des acides gras les plus longues (C10 à C14), d'où l'importance de l'utilisation de MCT d'origine végétale tels que proposés selon la présente invention, qui ont une composition allant dans ce sens. Les muscles candidats à l'injection d'une composition de MCT selon l'invention sont les muscles oxydatifs ou mixtes (producteurs d'énergie) à l'exclusion des muscles purement glycolytiques (producteurs de glycogène). En effet, les muscles sont classés en fonction de leur activité enzymatique. A cet égard, on a montré que, chez les bovins, les enzymes du métabolisme oxydatif sont représentatives de différentes voies métaboliques : l'hydrolyse des triglycérides circulants (lipoprotéine lipase, LPL), le catabolisme des acides gras à chaîne longue jusqu'à l'acétyl-CoA (enzymes de la béta-oxydation), le cycle de Krebs impliqué dans le catabolisme de l'acétyl-CoA produit à partir des acides gras ou du glucose (SDH, ICDH, etc.) ou la chaîne respiratoire intervenant dans la synthèse d'énergie (cytochrome c oxydase). La cinétique de différentiation musculaire décrite chez le bovin est proche de celle de l'humain comparativement à celle d'autres mammifères (B. Picard et al., INRA Prod. Anim. 2003, 16(2), 125-131).

En résumé, l'apport énergétique à la cellule augmente grâce au métabolisme des acides gras et du glycérol. La masse injectée provoque en outre l'écartement des fibres musculaires. L'aponévrose s'assouplit, ce qui permet l'extension du muscle. Les fibres musculaires restent écartées en permanence, comme en situation d'exercice physique.

Des pré-études échographiques montrent la semi-permanence du volume musculaire acquis, même après métabolisation du produit injecté. Il s'agit d'une hypertrophie en ce sens que le volume des fibres musculaires a augmenté.

### Procédé de fabrication

Le procédé de fabrication de la composition injectable selon l'invention comprend au moins les étapes suivantes :
- pesée et mélange éventuel des matières premières (MCT, alcool benzylique et tocophérol) ;
- contrôle de la concentration en alcool benzylique ; si la concentration est « Ok », passer à l'étape suivante, sinon retour à l'étape précédente ;
- filtration sur filtre stérilisant de 0,22 µm ;
- stockage intermédiaire dans des récipients de contention;
- distribution dans des ampoules de 5 mL, préalablement chauffées à 220°C et refroidies à l'air stérile ; élimination des ampoules excédentaires ;
- chauffage en autoclave à 120°C pendant 15 à 20 minutes ;
- contrôle à 100% des ampoules pour recherche de fuites, particules, fissures, opalescence (entrée d'eau) ; si contrôle « Ok », passer à l'étape suivante, sinon éliminer les ampoules non conformes ;
- emballage primaire (plaques) ;
- emballage secondaire (boîtes) ;
- stockage en quarantaine ;
- contrôle et approbation des lots ; si « Ok », expédier à l'utilisateur, sinon écarter les lots défectueux.

Durant le procédé de fabrication, les normes GMP et les spécifications d'assurance qualité sont appliquées.

### Domaines d'application

La présente invention trouve avantageusement des applications de traitement dans les domaines suivants :
- maladies neurodégénératives (ex. maladie de Charcot - lutte contre l'amyotrophie) ;
- polyomélite (rééquilibrage musculaire des deux jambes par exemple) ;
- SIDA (traitement des esquarres liés à la perte musculaire au niveau fessier) ;
- domaine post-traumatique (par exemple traitement de la perte musculaire liée à la pose d'un plâtre) ;
- domaine psychologique (par exemple dans le cas d'une personne complexée d'avoir des mollets petits ou trop fins) ;
- domaine sportivo-esthétique, catch, bodybuilding, strip-tease, etc. (rééquilibrage, recherche d'harmonie musculaire).

## Revendications

1. Utilisation d'une préparation injectable en intra-musculaire et bio-résorbable dans l'organisme humain ou animal, se présentant sous la forme d'une solution huileuse stérilisée comprenant :
- des triglycérides à chaîne moyenne (MCT), de préférence d'origine végétale,
- optionnellement de l'alcool benzylique,
- optionnellement de l'alpha-tocophérol,
pour la réalisation d'un implant musculaire destiné au traitement de maladies neurodégénératives d'atrophie musculaire ou au traitement des séquelles de la polyomélite ou en vue de l'obtention d'un rééquilibrage ou d'une harmonie musculaire à des fins esthétiques ou à des fins de traitement post-traumatique bu psychologique, ou encore pour la réalisation d'un implant fessier destiné au traitement de la perte musculaire liée au SIDA.

2. Utilisation selon la revendication 1, **caractérisé en ce que** lesdits MCT contenus dans ladite préparation sont d'origine végétale et proviennent de l'estérification avec du glycérol d'une composition d'acides gras contenant presque 100% d'acide caprylique (C8) et d'acide caprique (C10).

3. Utilisation selon la revendication 1 ou 2, **caractérisée en ce que** lesdits MCT contenus dans ladite préparation sont d'origine végétale et proviennent de l'estérification de la composition d'acides gras suivants :
- acide capronique (C6) : < 2% ;
- acide caprylique (C8) : 50 - 80% ;
- acide caprique (C10) : 20 -50% ;
- acide laurique (C12) : < 3% ;
- acide myristique (C14) : < 1%.

4. Utilisation selon la revendication 1, 2 ou 3, **caractérisée en ce que** lesdits MCT contenus dans ladite préparation sont obtenus par fractionnement d'huile de noix de coco et/ou de palme.

5. Utilisation selon l'une quelconque des revendications précédentes, **caractérisée en ce que** ladite préparation est bio-résorbable en une période de 30 jours.

## Patentansprüche

1. Verwendung einer intramuskulär injizierbaren und im Organismus eines Menschen oder Tiers biologisch resorbierbaren Zubereitung, die sich in Form einer sterilisierten öligen Lösung darstellt, die umfasst:
- mittelkettige Triglyceride (MCT), vorzugsweise pflanzlichen Ursprungs,
- optional Benzylalkohol,
- optional alpha-Tocopherol,
zum Erhalten eines Muskelimplantats, das zur Behandlung neurodegenerativer Muskelatrophieerkrankungen oder zur Behandlung der Folgen der Polyomyelitis oder zwecks Erhalt eines Muskelausgleichs oder einer Muskelharmonisierung aus ästhetischen Zwecken oder aus Zwecken der posttraumatischen oder psychologischen Behandlung oder auch zum Erhalten eines Gesäßimplantats bestimmt ist, das zur Behandlung von Muskelschwund bei AIDS bestimmt ist.

2. Verwendung nach Anspruch 1, **dadurch gekennzeichnet, dass** die in der Zubereitung vorhandenen MCT pflanzlich sind und von der Veresterung einer Fettsäurezusammensetzung, die fast 100 % Caprylsäure (C8) und Caprinsäure (C10) enthält, mit Glycerol herrühren.

3. Verwendung nach Anspruch 1 oder 2, **dadurch gekennzeichnet, dass** die in der Zubereitung vorhandenen MCT pflanzlich sind und von der Veresterung der folgenden Fettsäurezusammensetzung herrühren:
- Capronsäure (C6): < 2 %,
- Caprylsäure (C8): 50 - 80 %,
- Caprinsäure (C10): 20 - 50 %,
- Laurinsäure (C12): < 3 %,
- Myristinsäure (C14): < 1 %.

4. Verwendung nach Anspruch 1, 2 oder 3, **dadurch gekennzeichnet, dass** die in der Zubereitung enthaltenen MCT durch Fraktionierung von Kokosnuss- und/oder Palmöl hergestellt werden.

5. Verwendung nach einem der vorangehenden Ansprüche, **dadurch gekennzeichnet, dass** die Zubereitung in einem Zeitraum von 30 Tagen biologisch resorbierbar ist.

## Claims

1. Use of a bioresorbable preparation for intramuscular injection in a human or animal body, appearing as a sterilized oily solution comprising:
- medium chain triglycerides (MCTs), of plant origin preferably,
- optionally benzyl alcohol,
- optionally alpha-tocopherol,
for obtaining a muscle implant intended for treating muscular atrophy neurodegenerative diseases or for treating the after-effects of poliomyelitis or with a view to obtain a muscular rebalancing or harmony for aesthetic purposes or for post-traumatic or psychological treatment purposes, or further for obtaining a buttock implant intended for treating the muscle loss related to AIDS.

2. Use according to claim 1, **characterized in that** said MCTs contained in said preparation are of plant origin and stem from the esterification with glycerol of a composition of fatty acids containing almost 100% of caprylic acid (C8) and of capric acid (C10).

3. Use according to claim 1 or 2, **characterized in that** said MCTs contained in said preparation are of plant origin and stem from the esterification of the composition of the following fatty acids:
- capronic acid (C6): < 2%;
- caprylic acid (C8): 50-80%;
- capric acid (C10): 20-50%;
- lauric acid (C12): < 3%;
- myristic acid (C14): < 1%.

4. Use according to claim 1, 2 or 3, **characterized in that** said MCTs contained in said preparation are obtained by a fractionation of coconut and/or palm oil.

5. Use according to any of the preceding claims, **characterized in that** said preparation is bioresorbable within a period of 30 days.
